# EUROPEAN PATENT SPECIFICATION

(11) **EP 1 926 470 B1**
(45) Date of publication and mention of the grant of the patent: **04.07.2012**
(21) Application number: 06707207.4
(22) Date of filing: 23.02.2006
(51) Int. Cl.: A61K 8/894, A61K 8/891, A61Q 1/02

(54) **PREPARATION, IN PARTICULAR COSMETIC PREPARATION, PRODUCTION AND USE THEREOF**
ZUBEREITUNG, INSBESONDERE KOSMETISCHE ZUBEREITUNG, IHRE HERSTELLUNG UND VERWENDUNG
PRÉPARATION, PRÉPARATION COSMÉTIQUE EN PARTICULIER, PRODUCTION ET UTILISATION DE CELLE-CI

(30) Priority: 22.09.2005 DE 102005045353
(43) Date of publication of application: 04.06.2008
(73) Proprietor: Schwan-STABILO Cosmetics GmbH & Co. KG, 90562 Heroldsberg (DE)
(72) Inventor: PINZER, Reinhard, 91220 Schnaittach (DE); SPROGAR, Christian, 91088 Bubenreuth (DE)
(74) Representative: Leissler-Gerstl, Gabriele
(86) International application number: PCT/EP2006/001650
(87) International publication number: WO 2007/038993

(56) References cited:
- EP-A- 1 358 866
- DE-A1- 10 311 302
- US-A1- 2005 118 124
- PATENT ABSTRACTS OF JAPAN vol. 2000, no. 12, 3 January 2001 (2001-01-03) & JP 2000 239119 A (SHISEIDO CO LTD), 5 September 2000 (2000-09-05) cited in the application
- PATENT ABSTRACTS OF JAPAN vol. 2003, no. 12, 5 December 2003 (2003-12-05) & JP 2005 232069 A (SHISEIDO CO LTD), 2 September 2005 (2005-09-02)

## Description

The present invention concerns a preparation, in particular a cosmetic preparation, and a process for the production thereof.

The preparation according to the invention is in an emulsiontype form. The use of W/O or W/Si emulsions in the field of cosmetics is known. Silicones are readily used for cosmetic compositions by virtue of their non-transfer properties as the finished products can be workably applied, they present good adhesive strength and they stay in place. It will be noted however that the high water content which is inherent in those Known compositions means that they frequently suffer from the disadvantage that they cause an unpleasant burning cooling effect. The products in the state of the art also suffer from the disadvantage that they run and spread due to moisture, for example perspiration or tears. In order to avoid that film-forming agents are added to the formulations. That however can result in sticky products which cause a taut feeling on the skin.

In order to impart structure to the composition, waxes and wax-like compounds are often added, which suffer from the disadvantage that the film produced is matt. In addition waxes are generally high-melting so that relatively high temperatures have to be applied for processing them. That causes difficulties in terms of processing silicone-bearing compositions as in general volatile silicones must be used as a solvent, which have a low flash point, below 80°C. Therefore increased safety precautions have to be taken for processing such compositions.

A further problem with cosmetic compositions which contain a high proportion of volatile and non-volatile silicones is that, although they can be satisfactorily applied and afford good adhesion, after evaporation of the volatile solvent there is a feeling of tautness on the skin, which is perceived as being unpleasant.

JP 2001 058921 discloses for example a water-in-oil emulsion which enjoys excellent stability and which contains a specific silicone, oil, water, ethyl alcohol and dextrin fatty acid ester. The proportion of water and ethyl alcohol is very high in those emulsions. Such a high proportion of ethanol can result in irritation to the skin and in particular the mucous membranes. Furthermore aqueous systems of that kind can suffer from microbial contamination. In order to avoid contamination it is necessary to add preserving agents which, particularly in conjunction with ethanol, can give rise to fresh problem in relation to worse skin compatibility.

In addition a cosmetic preparation is known from JP 2000 0239119, which can contain a pearl pigment, wherein the emulsion is made up of a silicone compound in combination with a low alcohol and waster. The proportion of water and alcohol is also high with that product.

A gel-like product is described in JP 09194323, which also has a very high proportion of water. That product can be processed to afford makeup products such as eyeshadow and lipstick as well as sun protection cream. What is common to all those compositions is that a polyether-modified silicone is used as an emulsifier. The proportion of water and alcohol is high in all those compositions.

It is further known from US No. 2004/0028633 A1 to use a water-in-oil emulsion with a high proportion of inner aqueous phase for which special emulsifiers and salts are used for stabilisation purposes. The emulsifier proposed is a combination of two different polyether-modified silicones of different molecular weights, which stabilise an outer oil phase while the salt is intended to stabilise the inner aqueous phase. The proportion of water can be up to 80% here.

EP 0 568102 discloses a silicone gel composition comprising silicone oil, water and an emulsifier, wherein the emulsifier is a polyether-modified silicone which has 25 - 30 ethoxy and propoxy units respectively.

A disadvantage of the known compositions is their high water content which, besides the microbiological problems, results in a markedly cooling effect. Unlike the situation with the products referred to as cold creams which are distributed over the face and the body over a large area and in respect of which a cooling effect is desired, the cooling effect when applied over a small area, in particular in the proximity of the eyes, is perceived as being a nuisance and even an irritation.

The object of the invention was to improve the known compositions and in particular to provide compositions which can be applied to skin and mucous membranes, in particular eyelids and lips, without producing a disturbing and tautness feel there, which do not lose their original flexibility and stretchability, which cling firmly and stay in place, which can be easily removed and which remain stable over a prolonged period of time. The invention further aims to provide products which afford as desired semi-matt, silk-matt, shiny or highly shiny films.

Those objects are attained by a preparation as defined as claim 1.

The preparation according to the invention contains the following components:
a) at least one silicone;
b) at least one PEG/PPG dimethicone with respectively 15 to 20 units of PEG and PPG as emulsifier;
c) a lipid phase which can have oil, fat and/or wax;
d) a polar liquid in a proportion of 0.1 to 5% with respect to the total weight of the composition; and
e) at least one particulate ingredient.

This preparation is in a form wherein the polar fluid or liquid is distributed in the composition in homogenously and finely divided. The preparation can be in the form of an emulsion or a gellike composition wherein the polar liquid is finely distributed in the less polar ingredients. The emulsifier can contribute to the fine distribution.

Further ingredients can be added as required in order to modify the properties of the product.

It was surprisingly found that a preparation of the claimed form which contains a small amount of polar liquid in finely distributed form, which in one embodiment can be in the form of a W/O emulsion or a W/Si emulsion has the properties which are advantageous for silicones, without producing a feeling of tautness. In addition the preparation according to the invention remains stable over a long period of time. It was found that, when the preparation according to the invention is applied to the skin, it is not only not carried away by moisture which is present or occurs at the point of application or in the very close proximity thereof, such as perspiration, tears and so forth, but even stabilised thereby. That effect however can occur only if the preparation already contains a small proportion of water or other polar substances such as for example monovalent or polyvalent alcohols. Without being tied down to a theory it is assumed that, in a preparation of the claimed nature, the small proportion of a polar substance leads to gel formation and that the gel structure formed additionally stabilises polar substances, particularly water incorporated into the preparation. In the case of the products according to the invention that achieves the advantages attained with the silicones and at the same time avoids the problems which are otherwise linked thereto. Those advantageous properties are achieved only when the preparation already contains a polar substance, in particular water, although only in a small proportion. A preparation which is free from polar solvents, after application to the skin, after evaporation of the solvent, leads to a feeling of tautness, while preparations with a higher proportion of water entail the above-discussed disadvantages.

In addition it was found that the preparation according to the invention exhibits a pronounced thixotropy behaviour. Thixotropes or intrinsically viscous properties are highly advantageous for that kind of compositions as the preparation can be introduced into containers in pasty form and does not run out of the container, but it is very workable upon being applied and therefore can be pleasantly applied in a thin layer. The advantageous properties of the protection according to the invention are afforded by the use of a special combination of emulsifier and silicone, beside the low proportion of a polar liquid in particular water.

The silicone component of the preparation according to the invention can be formed by volatile or non-volatile silicones. Preferably at least one volatile silicone is included in the preparations according to the invention. Volatile silicones are known to the man skilled in the art in this field, for example volatile cyclomethicones and volatile linear dimethicones. Examples of volatile cyclomethicones are cyclic siloxanes with 3 to 7 dimethyl siloxane units such as cyclotrisiloxane (hexamethyl cyclotrisiloxane), cyclotetrasiloxane (octamethyl cyclotetrasiloxane), cyclopentasiloxane (decamethyl cyclopentasiloxane), cyclohexasiloxane (dodecamethyl cyclohexasiloxane). Examples of linear silicones are linear siloxanes with 3 to 10 dimethyl siloxane units such as hexamethyl disiloxane, octamethyl trisiloxane, decamethyl tetrasiloxane, dodecamethyl pentasiloxane or mixture of the individual substances, wherein both mixtures of various linear silicone oils, various cyclomethicones and also mixtures of cyclic and linear silicones are suitable. Usually those compounds which have a boiling point below 200°C are viewed as volatile silicones.

The preparation according to the invention, in addition to the volatile silicone, may also include a silicone which is not volatile at body temperature and at ambient temperature. Silicone oils and silicone resins can be considered here, and examples in this respect are well known to the man skilled in the art in the field of cosmetics. Silicone oils and silicone resins which are suitable for cosmetic preparations are available in a great variety and are commercially obtainable. Silicone oils in the form of long-chain dimethyl siloxanes and branched siloxanes which are also referred to silicone resins can be named as examples. Products such as for example dimethicone, phenyl trimethicone, diphenyl dimethicone and alkyl dimethicone can be used as non-volatile silicone oils. Mixtures of different silicone compounds are also suitable. A preferred class of silicones is compounds with 3 siloxane units of which the middle siloxane unit carries a longer-chain alkyl residue while the other valencies are saturated by methyl groups. That kind of compounds is commercially available under the INCl name trisiloxane or trimethicone. Caprylyl trimethicone, lauryl trimethicone and stearyl trimethicone as well as mixtures thereof can be preferably mentioned here.

The use of silicone emulsifiers for this type of W/Si emulsions which are suitable for that purpose are basically known. Polyether-modified silicones, as are described for example in EP 0 568 102, have proven to be particularly useful emulsifiers. That kind of emulsifiers has a long chain of dimethyl siloxane units which carry polyethoxy-polypropoxy units in the chain and at the chain ends. The number of units and the length of the units have an influence on the properties. It was now found that in particular PEG/PPG dimethicones which respectively have 15 to 20 ethoxy and 15 to 20 propoxy units are considered for the present invention. That kind of emulsifiers is known and is commercially available. Mention is to be made by way of example of the products marketed by DOW CORNING under the INCI names cyclopentasiloxane PEG/PPG-18/18 dimethicone and cyclopentasiloxane PEG/PPG-19/19 dimethicone. Those emulsifiers are usually employed dissolved in a volatile siloxane, as was described hereinbefore.

As a further essential ingredient the preparation according to the invention includes a lipid phase as is usual in the field of cosmetics. The lipid phase can be formed from oils, fats, straight-chain or branched-chain, saturated or singly or multiply unsaturated fatty alcohols, wax alcohols and fatty acids, the esters thereof as well as waxes and wax esters in liquid or solid form. The individual constituents can be of vegetable, animal, mineral or synthetic origin. The term "liquid" relates in that case to substances which are in a form capable of flow in a temperature range of between 0 and 45°C. Examples of lipid components are set out hereinafter without wishing to limit the invention thereby. The substances are identified hereinafter with the "INCI name" which is usual in this field and with which the man skilled in the art is familiar:

Vegetable oils and hydrated vegetable oils such as for example Buxus chinensis oil (jojoba oil), hydrogenated jojoba oil, cottonseed oil, hydrogenated cottonseed oil, vegetable oil, hydrogenated vegetable oil, rapeseed oil, hydrogenated rapeseed oil, castor oil, hydrogenated castor oil, hydrogenated coco-glycerides and the like, as well as Magnifera indica (mango seed oil), Limnanthes alba (meadowfoam seed oil), Buty-rosperum parkii (shea butter), coconut oil, cocoa butter, Macadamia temifolia nut oil (Macadamia nut oil), isopropyl myristate, isopropyl palmitate, isopropyl isostearate, butyl stearate, isopropyl isostearate, isodecyl neopentanoate, oleyl erucate, oleyl
oleate, diethyl sebacate, hexyl laurate, PPG-5-laureth-5, ethylhexyl dodecanol, capryl/capric acid triglyceride, paraffin oils or mixtures thereof. In that respect the amounts used are desirably to be kept low in order to maintain good adhesion after evaporation of the volatile constituents. Proportions below 20% are preferred. Levels of concentration of use of below 5% are particularly preferred.

Suitable fatty alcohols, fatty acids or wax alcohols are cetyl alcohol, stearyl alcohol, behenyl alcohol or mixtures thereof, palmitic acid, stearic acid, behenic acid, melissic acid or mixture thereof. The waxes are selected from waxes of animal, vegetable, mineral or synthetic origin or mixtures and hybrids thereof. The waxes used are of a hardness of 2 to 40, the hardness value being determined by the needle penetration method. The operation of determining the hardness value is implemented in accordance with the American standard ASTM D5: at a temperature of 25°C a needle having a defined cone and weighing 2.5g which is loaded with a weight of 47.5g is caused to penetrate into a flat surface of a sample body. The depth of penetration is determined in tenths of millimetre after 5 seconds.

Beeswax, modified beeswax or so-called "Cera Bellina", lanolin wax, Japan wax, candelilla wax, ouricuri wax, carnauba wax, rice wax, flower waxes or fruit waxes such as for example orange flower wax, orange wax, jasmin wax or apple wax, montan wax, microcrystalline wax, paraffin wax, ozocerite, polyethylene waxes, waxes produced in accordance with the Fischer-Tropsch method, silicone waxes, long-chain esters such as for example myristyl myristate, cetyl palmitate, stearyl stearate, behenyl stearate, behenyl behenate, hydrated jojoba oil or mixtures thereof and their hybrids. The amounts used of such waxes or the mixtures thereof and/or their hybrids are generally in the range below 20 % by weight, preferably below 5 % by weight.

In order to impart the particular properties to the invention, as an essential component a polar liquid like water or a polar solvent or a mixture of water and polar solvents is included in a small proportion of only 0.1 to 5 % by weight. It was found that surprisingly the addition of a small amount of a polar solvent has the result that a film is formed, which is not smudged or caused to run by water but is stabilised. It is assumed that a gel-like consistency is produced which, without altering the other properties such as adhesiveness, fixedness, flexibility and malleability, can absorb water from the surroundings and thereby surprisingly stabilises the structure.

The preparation according to the invention therefore contains water or another polar solvent such as ethanol, propylene-1,2-glycol, dipropylene glycol, tripropylene glycol, glycerine, diglycerine, triglycerine, butylene glycols (for example butane-1,2-, - 1,3-, -1,4-diol), hexylene glycols (for example hexane-1,2-, -1,3-, -1,4- or -1,6-diol), trimethylpentane diol, polyethylene glycols of molecular weights in the range of 200 to 2000 Daltons or mixtures thereof with water. It is essential for the invention that the proportion of polar liquid does not exceed 5% with respect to the finished composition. Preferably the polar liquid is used in a proportion of 0.5 to 3%, in particular 0.8 to 2%. If the content of the polar liquid is too low, that is to say it is bellow 0.5%, the desired stabilisation effect is no longer achieved. An amount above 5% does not contribute any further advantageous properties but leads to the above-listed disadvantages.

The preparation according to the invention comprising special silicones and emulsifiers in combination with a small proportion of a polar liquid, particularly water, is highly suitable for absorbing solid particulate constituents and stabilising them in the structure form. The preparation according to the invention therefore contains particulate ingredients as a further component. The proportion of the particulate ingredients is adjusted in accordance with the desired consistency and effect of the material. In particular colouring agents, effect agents and fillers or thickening agents are used as the particulate ingredients. In that respect the proportion of the colouring agents and the effect agents depends on the desired colour and effect while thickening agents and fillers serve to adjust consistency.

In accordance with the invention in particular finely divided pigments are used as the colouring agents, and can optionally also be coated. Examples of inorganic and organic pigments are inter alia: titanium dioxide, zinc oxide, iron oxides, chromium oxide, chromium hydroxide, ultramarine, Berlin Blue (ferric blue), mica, pearlescence agents such for example titanium dioxide-coated mica, coloured mica coated with titanium dioxide and metal oxides, bismuth oxychloride, coated bismuth oxychloride, flake-form metal powder of aluminium, brass, bronze, copper, silver, gold which can optionally also be coated with coloured metal oxides, so-called "glitter" in the form of thin and finely divided plastic flakes of polyester or the like immobilised colouring agents and lakes of organic colouring agents with aluminium, zirconium, barium, calcium or strontium. That list is only given by way of example and is not definitive. Those additions generally depend on the extent to which they are allowed by the respective national or regional cosmetics legislation.

The colouring agents are preferably selected from covering to strongly covering colouring agents of mean particle sizes of between 200 nm and 5 µm in the case of the spherical colouring agents in powder form and/or from flake colouring agents with a mean particle size of between 5 and 145 µm, in particular between 10 and 30 µm. Markedly larger particle sizes can also be used to achieve particular effects, for example glitter effects. In that respect the quantitative proportions of pigments are usually in a range of 0.1 to 30 % by weight, preferably in a range of 3 to 15 % by weight and quite particularly preferably in a range of 5 to 10 % by weight. If the preparation according to the invention is used as a light projection agent, in particular as a care lip product, it is also possible to add thereto, besides those selected from the aforementioned pigments, as base colouring, cerium oxide, titanium dioxide and/or zinc oxide in the form of so-called nanopigments involving particle sizes in the range of between 5 and 25 nm in an amount of 1 to 10 % by weight and preferably 3 to 8 % by weight, optionally also in combination with usual UV-A and UV-B filter substances which are allowed by the respective cosmetics legislation.

In particular iridescent, fluorescent, phosphorescent, glittering and shimmering constituents as are known to the man skilled in the art in this field are used as the effect agents. Those effect agents are frequently used in the form of small balls, on the surface of which are immobilised compounds which provide the desired effect. Compounds which light up in long-wave UV light, so-called black light, are also considered here.

The particulate colouring agents have an influence on the consistency of the material. If necessary the consistency can additionally be adjusted by the addition of per se known thickening agents and fillers, in the desired range. By way of example fillers such as talcum, kaolin, aluminium oxide, silicon oxide, each in finely divided and amorphous form, starch and modified starch, polytetrafluoroethylene powder (Teflon), nylon powder and boronitride are suitable for that purpose. Thickening agents such as hectorite, bentonite, montmorrillonite and the chemically modified forms thereof are further suitable as thickening agents. The thickening agents are preferably modified in the form of insoluble metal soaps, for example as stearates, to make them more compatible.

In order to improve the processability of the particulate ingredients, in particular the fillers and thickening agents, they are preferably pre-treated prior to incorporation into the preparation with a volatile solvent, for example a solvent as can also be used for incorporation of the emulsifier. In a preferred embodiment the particulate constituents, prior to further processing, are mixed with a part of the volatile solvent so that wetting takes place.

In addition to the above-mentioned components which are essential to the invention the preparation according to the invention can contain further ingredients which are usual in the cosmetics field. Ingredients which are frequently employed are perfumes, individually and in combination, flavourings, anti-oxidants, preserving agents, care ingredients and so forth. Those ingredients can be added to the composition in per se known manner and in the usual amounts.

The preparation according to the invention can have a pasty consistency whose viscosity under the action of a shearing force changes in such a way that it can be very easily distributed on the skin or mucous membrane. Preferably the viscosity of the preparation is adjusted to a range of 800 to 5000 Pa·s, preferably 1100 to 3500 Pa·s. In that case the viscosity is determined with a commercially available rheometer with a sand-blasted plate/plate measuring device involving a plate diameter of 25 mm. Because of pronounced normal stressing under shear the preparations can only be reproducibly tested under highly dynamic measurement conditions. The measurement values are ascertained at a shearing rate of γ = 1/s after eight seconds at 25°C.

The preparations according to the invention are in the form of a soft malleable paste which can be easily and uniformly applied and distributed. It can be removed from the skin again in a manner known to the users - by suitable makeup removers or cloths or by washing with fine soap or suitable mild tenside preparations. It can be introduced in known manner into suitable receptacles such as flasks, possibly with a spatula, pots or tubes and can be taken therefrom again by the user. However, because of the improved hygiene conditions which are linked thereto, it can also be introduced into suitable applicator devices, referred to as dispenser mechanisms, and applied therefrom. Applicator devices as are known for example from US No 6 238 117 or US No 6 309 128 are suitable for the application of small amounts as are required for example for application in the lip or eye region, as such devices permit fine metering.

In a preferred embodiment the preparation according to the invention includes inter alia a proportion of a volatile silicone with a low flash point. As stated above the use of the specific emulsifier means that the preparation can be processed at low temperatures, which is of particular advantage in regard to safety. A further subject-matter of the present invention is therefore a process for the production of a preparation, as is defined in the claims, wherein the emulsifier is dissolved in a volatile solvent, the particulate ingredients are added, then the lipid phase and the further ingredients are added and homogenisation is effected, all process steps being carried out at ambient temperature, then the batch is deaerated by the application of vacuum and then-the polar liquid, for example water and optionally silicone are added.

A particular advantage of the process according to the invention is that it can be carried out at ambient temperature, in which case nonetheless a homogenous product with a pleasant texture is obtained.

In accordance with the invention there is provided a preparation which, by virtue of its components, has non-transfer properties and, by virtue of the content of volatile constituents, can also be applied in a highly malleable fashion, which has very good adhesion and remains in place and does not migrate into the fine wrinkles in the skin. In addition the preparation according to the invention after being applied can absorb further moisture from the surroundings without the properties thereof being adversely affected thereby. In contrast stability and strength are even enhanced by virtue of the absorption of water. That can be demonstrated by rising viscosity values upon the absorption of moisture. It was surprising in that respect that this preparation which is silicone-bearing and thus by virtue of its very type strongly hydrophobic - water-repellent - is capable of additionally absorbing moisture from the outside and being stabilised thereby, without in that case losing malleability. When applied to eyelids or lips the preparations according to the invention thus also do not produce any unpleasant or strange feel to the skin but are improved in terms of their positive properties without losing their original flexibility and stretchability.

The invention will now be described with reference to some Examples without however being limited thereby. In that respect in an individual case the amounts involved may certainly be slightly higher than or less than the foregoing quantitative values and in that case nonetheless preparations according to the invention are still obtained. The raw materials used in the Examples are identified by the INCI names (International Nomenclature of Cosmetic Ingredients) which are known to the man skilled in the relevant art. The quantities are stated in percent by weight with respect to the total weight of the preparation.

### Example 1 (Pearl lip colour)

| | | |
|---|---|---|
| PEG/PPG-19/19 dimethicone | | 16.000 |
| Cyclopentasiloxane | | 14.800 |
| Trisiloxane | | 38.050 |
| Polyethylene (micronized) | | 1.800 |
| Carnauba (micronized) | | 2.200 |
| Mica and titanium dioxide (CI-No 77.891) | | 11.000 |
| Iron oxides (CI-No 77.491, CI-No. 77.492 and CI-No 77.499) | | 1.300 |
| Silica | | 4.800 |
| Gel A: | cyclopentasiloxane and disteardimonium hectorite | 5.000 |
| Gel B: | isodecyl neopentanoate and cyclopentasiloxane and stearalkonium bentonite and propylene glycol and dimethicone/vinyl dimethicone crosspolymer | 3.000 |
| Phenoxyethanol | | 0.500 |
| Methylparaben | | 0.150 |
| Ethylparaben | | 0.025 |
| Propylparaben | | 0.075 |
| Fragrance | | 0.200 |
| Tocopherol | | 0.100 |
| Aqua/water | | 1.000 |

For the production procedure the PEG/PPG-19/19 dimethicone is dissolved in approximately half the cyclopentasiloxane in a vacuum process installation with intensive agitation, then the iron oxides, silica, micronized waxes and pre-produced gels A and B are added and the mixture is well homogenised at normal ambient temperature. The balance of the cyclopentasiloxane and the mica are now added and the mixture is thoroughly agitated without use of the homogeniser. The parabens and tocopherol are dissolved in the phenoxyethanol, optionally with heating, the fragrance mix is added and the mixture is added to the batch. That mixture is now deaerated by the application of vacuum with slight agitation. The trisiloxane is now added, the batch is homogenously agitated and gelled by the addition of the water. The result obtained is a strongly shiny, copper-coloured, highly viscous paste which nonetheless can be easily distributed on the skin, of a viscosity of 3,000 Pa·s.

### Example 2 (Pearl lip balm with light protection)

| | |
|---|---|
| PEG/PPG-19/19 dimethicone | 18.000 |
| Cyclopentasiloxane | 15.500 |
| Trisiloxane | 29.500 |
| Buxus chinensis oil | 3.500 |
| Butyrospermum parkii (shea butter) | 1.200 |
| Theobroma cacao (cocoa) seed butter | 1.500 |
| Euphorbia cerifera (candelilla) wax | 1.800 |
| Isopropyl isostearate | 2.000 |
| Mica and titanium dioxide (CI-No 77.891) | 10.000 |
| Titanium dioxide nanopigment (CI-No 77.891) | 5.000 |
| Iron oxides (CI-No 77.491) | 0.900 |
| Red 7 (CI-No. 15.850) | 0.450 |
| Silica | 4.800 |
| Cyclopentasiloxane and disteardimonium hectorite | 3.800 |
| Phenoxyethanol | 0.500 |
| Methylparaben | 0.150 |
| Ethylparaben | 0.025 |
| Propylparaben | 0.075 |
| Fragrance | 0.200 |
| Tocopherol acetate | 0.350 |
| Bisabolol | 0.100 |
| Retinyl palmitate | 0.150 |
| Aqua/water | 0.500 |

Production is effected in a similar manner to Example 1. The result obtained is a reddish, weakly covering, strongly pearlescent paste with a very good light protection effect, which can be easily applied to the lips and which does not tauten after drying and which does not transfer onto other articles. It has a viscosity of 1200 Pa·s.

### Example 3 (Pearl eyeshadow)

| | |
|---|---|
| PEG/PPG-19/19 dimethicone | 15.000 |
| Cyclopentasiloxane | 19.500 |
| Trisiloxane | 32.000 |
| Limnanthes alba (meadowfoam seed oil) | 2.500 |
| Butyrospermum parkii (shea butter) | 1.350 |
| Isostearyl isostearate | 1.500 |
| Mica and titanium dioxide (CI-No 77.891) | 12.500 |
| Ultramarlnes (CI-No 77.007) | 2.000 |
| Chromium hydroxide green (CI-No. 15.850) | 3.500 |
| Silica | 4.800 |
| Cyclopentasiloxane and disteardimonium hectorite | 3.800 |
| Phenoxyethanol | 0.500 |
| Methylparaben | 0.100 |
| Ethylparaben | 0.025 |
| Propylparaben | 0.075 |
| Fragrance | 0.100 |
| Tocopherol | 0.100 |
| Aqua/water | 0.650 |

Production is effected in a similar manner to Example 1. The result obtained is a turquoise-coloured, well-covering, strongly pearlescent paste which can be easily distributed on the eyelid and stays at the place of application for a very long time and does not tauten after drying. It is of a viscosity of 2400 Pa·s.

### Example 4 (blusher cream)

| | |
|---|---|
| PEG/PPG-19/19 dimethicone | 12.500 |
| Cyclopentasiloxane | 19.500 |
| Trisiloxane | 38.650 |
| Limnanthes alba (meadowfoam seed oil) | 3.500 |
| Butyrospermum parkii (shea butter) | 1.350 |
| Boron nitride | 2.800 |
| Isostearyl isostearate | 1.500 |
| Titanium dioxide (CI-No. 77.891) Iron oxides (CI-No. 77.491, CI-No. 77.492, | 4.500 |
| CI-No. 77.499) | 3.550 |
| Red 7 lake (CI-No. 15.850) | 0.350 |
| Kaolin | 1.500 |
| Silica | 4.800 |
| Isodecyl neopentanoate and cyclopentasiloxane and stearalkonium bentonite and propylene glycol and dimethicone/vinyl dimethicone crosspolymer | 3.700 |
| Phenoxyethanol | 0.500 |
| Methylparaben | 0.150 |
| Ethylparaben | 0.025 |
| Propylparaben | 0.075 |
| Fragrance | 0.200 |
| Tocopherol | 0.100 |
| Aqua/water | 0.750 |

Production is effected in a similar manner to Example 1. The result obtained is a matt, well-covering, bright paste which is red with a hint of brown, which can be very well distributed on the skin and permits the modelling of fluid transitions, which does not tauten on the skin after drying and which affords good adhesion. It is of a viscosity of 2800 Pa·s.

### Example 5 (hair gel which lights up under UV-light)

| | |
|---|---|
| PEG/PPG-19/19 dimethicone | 20.100 |
| Cyclopentasiloxane | 10.500 |
| Trisiloxane | 41.900 |
| Mica and titanium dioxide (CI-No. 77.891) | 14.500 |
| Polyethylene (micronized) | 4.000 |
| Silica | 3.850 |
| Isodecyl neopentanoate and cyclopentasiloxane and stearalkonium bentonite and propylene glycol and dimethicone/vinyl dimethicone crosspolymer | 2.500 |
| Phenoxyethanol | 0.500 |
| Methylparaben | 0.150 |
| Ethylparaben | 0.025 |
| Propylparaben | 0.075 |
| Disodium distyrylbiphenyl disulfonate and sodium chloride | 1.000 |
| Aqua/water | 0.900 |

Production is effected in a similar manner to Example 1. The result obtained is a slightly cloudy paste which can be very well applied to individual strands of the hair and which when irradiated with ultra-violet light, so-called black light (for example in a place for dancing) produces a bluish shimmer. It is of a viscosity of 2700 Pa·s.

## Claims

1. A preparation comprising
a) at least one silicone;
b) at least one PEG/PPG dimethicone with respectively 15 to 20 units of PEG and PPG as emulsifier;
c) a lipid phase;
d) a polar liquid in a proportion of 0.1 to 5% with respect to the total weight of the composition; and
e) at least one particulate substance.

2. A preparation according to claim 1 **characterised in that** a silicone which is volatile at ambient temperature and body temperature is used as the silicone.

3. A preparation according to claim 1 **characterised in that** a silicone which is non-volatile at ambient temperature and body temperature or a combination of a silicone which is volatile at ambient temperature and body temperature and a silicone which is non-volatile at ambient temperature and body temperature is used as the silicone.

4. A preparation according to one of the preceding claims **characterised in that** a silicone oil or silicone resin is used as the non-volatile silicone.

5. A preparation according to one of the preceding claims **characterised in that** C₈-C₂₀ alkyl trimethicone is included as the silicone.

6. A preparation according to one of the preceding claims **characterised in that** PEG/PPG-19/19 dimethicone is included as the emulsifier.

7. A preparation according to one of the preceding claims **characterised in that** a linear silicone with 2 to 20 dimethyl siloxane units or a cyclomethicone with 3 to 7 dimethyl siloxane units is included as the volatile silicone.

8. A preparation according to one of the preceding claims **characterised in that** hexamethyl cyclotrisiloxane, octamethyl cyclotetrasiloxane, decamethyl cyclopentasiloxane, dodecamethyl cyclohexasiloxane, hexamethyl disiloxane, octamethyl trisiloxane, decamethyl tetrasiloxane, dodecamethyl pentasiloxane or mixtures thereof are included as the volatile silicone.

9. A preparation according to one of the preceding claims **characterised in that** the lipid phase includes oils, fats, linear or branched saturated or singly or multiply unsaturated fatty alcohols, wax alcohols, fatty acids and their esters, waxes and wax esters or mixtures thereof.

10. A preparation according to one of the preceding claims **characterised in that** vegetable or hydrated vegetable oil is included as the oil.

11. A preparation according to one of the preceding claims **characterised in that** particulate colouring agents, effect agents and/or fillers are included as particulate ingredients.

12. A preparation according to one of the preceding claims **characterised in that** pigments in finely divided and/or amorphous form are included as colouring agents, which pigments can optionally be coated.

13. A preparation according to one of the preceding claims **characterised in that** glittering, shimmering, fluorescent, phosphorescent or iridescent agents or agents which light up under UV light are included as effect agents.

14. A preparation according to one of the preceding claims **characterised in that** fillers and thickening agents, optionally in chemically modified form, are additionally included.

15. A preparation according to one of the preceding claims **characterised in that** the particulate ingredients are included in the form of insoluble metal soaps.

16. A preparation according to one of the preceding claims **characterised in that** ingredients which are usual for cosmetic preparations are additionally included.

17. A preparation according to one of the preceding claims **characterised in that** perfume and perfume mixtures, flavourings, anti-oxidants, preserving agents and/or care active substance are additionally included.

18. A preparation according to one of the preceding claims **characterised in that** the finished preparation is of a viscosity of 800 to 5000 Pa·s at 25°C as measured on a commercially available rheometer with a sand-blasted plate/plate measuring device with a plate diameter of 25 mm.

19. A preparation according to one of the preceding claims **characterised in that** it is in the form of a pasty composition.

20. A preparation according to one of the preceding claims **characterised in that** it is in the form of a thixotropic paste.

21. A preparation according to one of the preceding claims **characterised in that** besides usual additives the preparations contain 12 to 21 % emulsifier, 10 to 25 % volatile silicone, 25 to 45 % non-volatile silicone, 2 to 10 % lipid phase, 2 to 10 % thickening phase, 5 to 20 % colouring agent and 0.1 to 5 % water.

22. A preparation according to claim 21 **characterised in that** suspension agents, preserving agents, fragrances, anti-oxidants and/or flavourings are included as usual additives.

23. A process for the production of a preparation according to one of claims 1 to 21 wherein the emulsifier is dissolved in a volatile solvent, the particulate ingredients are added, then the lipid phase and the further ingredients are added and homogenisation is effected, wherein all process steps are carried out at ambient temperature, then the batch is deaerated by the application of vacuum and then water and optionally silicone are added.

## Patentansprüche

1. Zubereitung enthaltend
a) mindestens ein Silikon;
b) mindestens ein PEG/PPG-Dimethicone mit jeweils 15 bis 20 Einheiten PEG und PPG als Emulgator;
c) eine Lipidphase;
d) eine polare Flüssigkeit in einem Anteil von 0,1 bis 5% bezogen auf das Gesamtgewicht der Zusammensetzung; und
e) mindestens einen teilchenförmigen Stoff.

2. Zubereitung nach Anspruch 1, **dadurch gekennzeichnet, dass** als Silikon ein bei Raumtemperatur und Körpertemperatur flüchtiges Silikon verwendet wird.

3. Zubereitung nach Anspruch 1, **dadurch gekennzeichnet, dass** als Silikon ein Silikon, welches bei Raumtemperatur und Körpertemperatur nichtflüchtig ist oder eine Kombination eines Silikons, welches bei Raumtemperatur und Körpertemperatur flüchtig ist und eines Silikons, welches bei Raumtemperatur und Körpertemperatur nichtflüchtig ist, verwendet wird.

4. Zubereitung nach einem der vorangehenden Ansprüche, **dadurch gekennzeichnet, dass** als nichtflüchtiges Silikon ein Silikonöl oder Silikonharz verwendet wird.

5. Zubereitung nach einem der vorangehenden Ansprüche, **dadurch gekennzeichnet, dass** als Silikon ein C₈-C₂₀-Alkyltrimethicone enthalten ist.

6. Zubereitung nach einem der vorangehenden Ansprüche, **dadurch gekennzeichnet, dass** als Emulgator PEG/PPG-19/19-Dimethicone enthalten ist.

7. Zubereitung nach einem der vorangehenden Ansprüche, **dadurch gekennzeichnet, dass** als flüchtiges Silikon ein lineares Silikon mit 2 bis 20 Dimethylsiloxaneinheiten oder ein Cyclomethicone mit 3 bis 7 Dimethylsiloxaneinheiten enthalten ist.

8. Zubereitung nach einem der vorangehenden Ansprüche, **dadurch gekennzeichnet, dass** als flüchtiges Silikon Hexamethylcyclotrisiloxan, Octamethylcyclotetrasiloxan, Decamethylcyclopentasiloxan, Dodecamethylcyclohexasiloxan, Hexamethyldisiloxan, Octamethyltrisiloxan, Decamethyltetrasiloxan, Dodecamethylpentasiloxan oder Mischungen daraus enthalten sind.

9. Zubereitung nach einem der vorangehenden Ansprüche, **dadurch gekennzeichnet, dass** die Lipidphase Öle, Fette, lineare oder verzweigte gesättigte oder einfach oder mehrfach ungesättigte Fettalkohole, Wachsalkohole, Fettsäuren sowie deren Ester, Wachse und Wachsester oder Mischungen davon enthält.

10. Zubereitung nach einem der vorangehenden Ansprüche, **dadurch gekennzeichnet, dass** als Öl pflanzliches oder hydriertes pflanzliches Öl enthalten ist.

11. Zubereitung nach einem der vorangehenden Ansprüche, **dadurch gekennzeichnet, dass** als teilchenförmige Inhaltsstoffe teilchenförmige Färbemittel, Effektmittel und/oder Füllstoffe enthalten sind.

12. Zubereitung nach einem der vorangehenden Ansprüche, **dadurch gekennzeichnet, dass** als Färbemittel Pigmente in feinteiliger und/oder amorpher Form enthalten sind, die gegebenenfalls beschichtet sein können.

13. Zubereitung nach einem der vorangehenden Ansprüche, **dadurch gekennzeichnet, dass** als Effektmittel glitzernde, schimmernde, fluoreszierende, phosphoreszierende oder irisierende Mittel oder Mittel, welche unter UV-Licht leuchten, enthalten sind.

14. Zubereitung nach einem der vorangehenden Ansprüche, **dadurch gekennzeichnet, dass** zusätzlich Füllstoffe und Verdickungsmittel, gegebenenfalls in chemisch modifizierter Form, enthalten sind.

15. Zubereitung nach einem der vorangehenden Ansprüche, **dadurch gekennzeichnet, dass** die teilchenförmigen Inhaltsstoffe in Form von unlöslichen Metallseifen enthalten sind.

16. Zubereitung nach einem der vorangehenden Ansprüche, **dadurch gekennzeichnet, dass** zusätzlich für kosmetische Zubereitungen übliche Inhaltsstoffe enthalten sind.

17. Zubereitung nach einem der vorangehenden Ansprüche, **dadurch gekennzeichnet, dass** zusätzlich Riechstoffe und Riechstoffgemische, Aromen, Antioxidantien, Konservierungsmittel und/oder Pflegewirkstoffe enthalten sind.

18. Zubereitung nach einem der vorangehenden Ansprüche, **dadurch gekennzeichnet, dass** die fertige Zubereitung eine Viskosität, gemessen auf einem handelsüblichen Rheometer mit einer sandgestrahlten Platte-Platte-Messeinrichtung mit einem Plattendurchmesser von 25 mm, bei 25°C 800 bis 5000 Pa s ist.

19. Zubereitung nach einem der vorangehenden Ansprüche, **dadurch gekennzeichnet, dass** sie als pastöse Zusammensetzung vorliegt.

20. Zubereitung nach einem der vorangehenden Ansprüche, **dadurch gekennzeichnet, dass** sie als thixotrope Paste vorliegt.

21. Zubereitung nach einem der vorangehenden Ansprüche, **dadurch gekennzeichnet, dass** die Zubereitungen neben üblichen Zusatzstoffen 12 bis 21% Emulgator, 10 bis 25% flüchtiges Silikon, 25 bis 45% nichtflüchtiges Silikon, 2 bis 10% Lipidphase, 2 bis 10% Verdickungsmittel, 5 bis 20% Färbemittel und 0,1 bis 5% Wasser enthalten.

22. Zubereitung nach Anspruch 21, **dadurch gekennzeichnet, dass** als übliche Zusatzstoffe Suspensionsmittel, Konservierungsmittel, Duftstoffe, Antioxidantien und/oder Aromen enthalten sind.

23. Verfahren zur Herstellung einer Zubereitung nach einem der Ansprüche 1 bis 21, wobei der Emulgator in einem flüchtigen Lösungsmittel aufgelöst wird, die teilchenförmigen Inhaltsstoffe zugefügt werden, dann die Lipidphase und die weiteren Inhaltsstoffe zugefügt werden und Homogenisierung bewirkt wird, wobei alle Verfahrensschritte bei Raumtemperatur ausgeführt werden, wobei dann der Ansatz durch Einsatz eines Vakuums entlüftet wird, und dann Wasser und gegebenenfalls Silikon zugefügt werden.

## Revendications

1. Préparation comprenant
a) au moins une silicone ;
b) au moins une diméthicone de PEG/PPG présentant respectivement de 15 à 20 unités de PEG et de PPG en tant qu'émulsifiant ;
c) une phase de lipide ;
d) un liquide polaire selon une proportion allant de 0,1 à 5 % par rapport au poids total de la composition ; et
e) au moins une substance particulaire.

2. Préparation selon la revendication 1, **caractérisée en ce qu'**une silicone qui est volatile à température ambiante et à température corporelle est utilisée en tant que silicone.

3. Préparation selon la revendication 1, **caractérisée en ce qu'**une silicone qui est non volatile à température ambiante et à température corporelle ou une combinaison d'une silicone qui est volatile à température ambiante et à température corporelle et d'une silicone qui est non volatile à température ambiante et à température corporelle est utilisée en tant que silicone.

4. Préparation selon l'une des revendications précédentes, **caractérisée en ce qu'**une huile de silicone ou une résine de silicone est utilisée en tant que silicone non volatile.

5. Préparation selon l'une des revendications précédentes, **caractérisée en ce qu'**une triméthicone d'alkyle en C₈ à C₂₀ est incluse en tant que silicone.

6. Préparation selon l'une des revendications précédentes, **caractérisée en ce qu'**une diméthicone de PEG/PPG-19/19 est incluse en tant qu'émulsifiant.

7. Préparation selon l'une des revendications précédentes, **caractérisée en ce qu'**une silicone linéaire présentant de 2 à 20 unités de diméthylsiloxane ou une cyclométhicone présentant de 3 à 7 unités de diméthylsiloxane est incluse en tant que silicone volatile.

8. Préparation selon l'une des revendications précédentes, **caractérisée en ce qu'**un hexaméthylcyclotrisiloxane, un octaméthylcyclotétrasiloxane, un décaméthylcyclopentasiloxane, un dodécaméthylcyclohexasiloxane, un hexaméthyldisiloxane, un octaméthyltrisiloxane, un décaméthyltétrasiloxane, un dodécaméthylpentasiloxane ou des mélanges de ceux-ci sont inclus en tant que silicone volatile.

9. Préparation selon l'une des revendications précédentes, **caractérisée en ce que** la phase de lipide inclut des huiles, des graisses, des alcools gras saturés ou insaturés une seule fois ou plusieurs fois, linéaires ou ramifiés, des alcools de cire, des acides gras et leurs esters, des cires et des esters de cire ou des mélanges de ceux-ci.

10. Préparation selon l'une des revendications précédentes, **caractérisée en ce qu'**une huile végétale ou végétale hydratée est incluse en tant qu'huile.

11. Préparation selon l'une des revendications précédentes, **caractérisée en ce que** des agents de coloration particulaires, des agents à effet et/ou des agents de remplissage sont inclus en tant qu'ingrédients particulaires.

12. Préparation selon l'une des revendications précédentes, **caractérisée en ce que** des pigments sous une forme amorphe et/ou finement divisée sont inclus en temps qu'agents de coloration, lesquels pigments peuvent éventuellement être revêtus.

13. Préparation selon l'une des revendications précédentes, **caractérisée en ce que** des agents à effet de paillettes, à effet de satiné, à effet fluorescent, à effet phosphorescent ou à effet irisé ou des agents qui se révèlent sous une lumière ultraviolette sont inclus en tant qu'agents à effet.

14. Préparation selon l'une des revendications précédentes, **caractérisée en ce que** des agents de remplissage et des agents d'épaississement, éventuellement sous une forme modifiée sur le plan chimique, sont en outre inclus.

15. Préparation selon l'une des revendications précédentes, **caractérisée en ce que** les ingrédients particulaires sont inclus sous la forme de savons métalliques solubles.

16. Préparation selon l'une des revendications précédentes, **caractérisée en ce que** des ingrédients qui sont usuels pour les préparations cosmétiques sont en outre inclus.

17. Préparation selon l'une des revendications précédentes, **caractérisée en ce qu'**un parfum et des mélanges de parfums, des aromatisants, des antioxydants, des agents de conservation et/ou une substance de soins active sont en outre inclus.

18. Préparation selon l'une des revendications précédentes, **caractérisée en ce que** la préparation finie présente une viscosité allant de 800 à 5 000 Pa.s à 25 °C telle que mesurée sur un rhéomètre disponible dans le commerce présentant un dispositif de mesure à plaque/plaque décapée au sable présentant un diamètre de plaque de 25 mm.

19. Préparation selon l'une des revendications précédentes, **caractérisée en ce qu'**elle se présente sous la forme d'une composition pâteuse.

20. Préparation selon l'une des revendications précédentes, **caractérisée en ce qu'**elle se présente sous la forme d'une pâte thixotrope.

21. Préparation selon l'une des revendications précédentes, **caractérisée en ce qu'**en plus des additifs usuels, les préparations contiennent de 12 à 21 % d'émulsifiant, de 10 à 25 % de silicone volatile, de 25 à 45 % de silicone non volatile, de 2 à 10 % de phase de lipide, de 2 à 10 % de phase d'épaississement, de 5 à 20 % d'agent de coloration et de 0,1 à 5 % d'eau.

22. Préparation selon la revendication 21, **caractérisée en ce que** des agents de mise en suspension, des agents de conservation, des fragrances, des antioxydants et/ou des aromatisants sont inclus en tant d'additifs usuels.

23. Procédé de production d'une préparation selon l'une des revendications 1 à 21, dans lequel l'émulsifiant est dissout dans un solvant volatile, les ingrédients particulaires sont ajoutés, puis la phase de lipide et les autres ingrédients sont ajoutés et une homogénéisation est effectuée, où toutes les étapes de procédé sont effectuées à température ambiante, puis le lot est désaéré par l'application d'un vide, puis de l'eau et éventuellement une silicone sont ajoutées.
